# FASCICULE DE BREVET EUROPEEN

(11) **EP 2 043 526 B1**
(45) Date de publication et mention de la délivrance du brevet: **24.02.2010**
(21) Numéro de dépôt: 07803841.1
(22) Date de dépôt: 04.07.2007
(51) Int. Cl.: A61B 10/02

(54) **BROSSE DE PRELEVEMENT CYTOLOGIQUE**
BÜRSTE FÜR ZYTOLOGISCHE PROBENENTNAHME
CYTOLOGICAL SAMPLING BRUSH

(30) Priorité: 26.07.2006 FR 0606856
(43) Date de publication de la demande: 08.04.2009
(73) Titulaire: NOVACYT, 78140 Velizy Villacoublay (FR)
(72) Inventeur: PELTIER, Eric, F-92140 Clamart (FR)
(74) Mandataire: Jacobson, Claude
(86) Numéro de dépôt international: PCT/FR2007/001135
(87) Numéro de publication internationale: WO 2008/012407

(56) Documents cités:
- EP-A- 0 448 137
- EP-A2- 0 283 272
- WO-A-99/53841

## Description

La présente invention concerne une brosse de prélèvement cytologique.

Plus particulièrement, l'invention se rapporte à une telle brosse qui comporte un manche de manipulation à l'une des extrémités duquel sont prévues une première portion en forme de brosse de prélèvement exocol et une seconde portion en forme d'écouvillon de prélèvement endocol au centre de la première portion en forme de brosse.

Les brosses de prélèvement actuellement connues dans l'état de la technique présentent un certain nombre d'inconvénients, car la portion en forme d'écouvillon n'est en général pas assez longue et trop souple pour assurer un prélèvement correct de cellules au niveau de l'endocol voire de la zone de jonction exocol-endocol.

Or, un prélèvement incorrect de cellules peut se traduire par le fait que l'analyse ultérieure de ces cellules sera incomplète avec pour conséquence le fait que cette analyse ne mettra pas en évidence un problème quelconque.

Le but de l'invention est donc de résoudre ces problèmes. EP0283272 divulgue les caracteristiques de la preamble de la revendication 1.

A cet effet, l'invention a pour objet une brosse de prélèvement cytologique du type comportant un manche de manipulation à l'une des extrémités duquel sont prévues une première portion en forme de brosse de prélèvement exocol et une seconde portion en forme d'écouvillon de prélèvement endocol au centre de la première portion en forme de brosse, caractérisée en ce que la seconde portion en forme d'écouvillon est fixée à l'extrémité du manche et est détachable de la première portion par coulissement dans un trou de la première portion en forme de brosse, muni de moyens de retenue de cellules permettant de recueillir des cellules de la seconde portion en forme d'écouvillon, lors de son retrait de la première portion en forme de brosse.

Selon d'autres caractéristiques de l'invention :
- la première portion en forme de brosse est fixée sur le manche par des moyens de fixation détachables;
- les moyens de fixation détachables comprennent des moyens de fixation à baïonnette ; et
- les moyens de retenue du trou de la première portion en forme de brosse comprennent des moyens en forme de peigne à travers lesquels passe la seconde portion en forme d'écouvillon lors de son retrait de la première portion en forme de brosse.

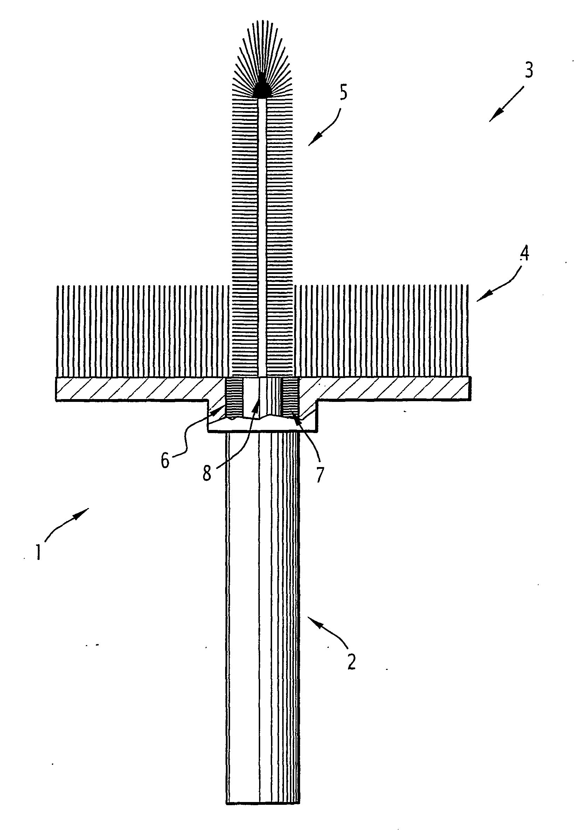

L'invention sera mieux comprise à la lecture de la description qui va suivre, donnée uniquement à titre d'exemple et faite en se référant au dessin annexé qui représente une vue schématique en coupe partielle d'une brosse de prélèvement cytologique selon l'invention.

On a en effet illustré sur cette figure, une brosse de prélèvement cytologique qui est désignée par la référence générale 1 et qui comporte un manche de manipulation désigné par la référence générale 2, à l'une des extrémités duquel sont prévus des moyens en forme de brosse désignés par la référence générale 3.

En fait, ces moyens comprennent une première portion en forme de brosse de prélèvement exocol désignée par la référence générale 4, et une seconde portion en forme d'écouvillon de prélèvement endocol désignée par la référence générale 5 et prévue par exemple au centre de la première portion en forme de brosse 4.

Selon l'invention, la seconde portion en forme d'écouvillon désignée par la référence générale 5 est fixée à l'extrémité du manche 2 et est détachable de la première portion par coulissement dans un trou désigné par la référence générale 6 de cette première portion en formé de brosse. Ce trou 6 est muni de moyens 7 de retenue de cellules permettant de recueillir des cellules de la seconde portion en forme d'écouvillon, lors de son retrait par coulissement dans ce trou de la première portion en forme de brosse.

Les moyens de retenue 7 peuvent en fait être constitués par des moyens en forme de peigne permettant de retenir les cellules lors du retrait de la seconde portion en forme d'écouvillon

On notera également que la première portion en forme de brosse peut être fixée sur le manche 2 par l'intermédiaire de moyens de fixation détachables désignés par la référence générale 8 sur cette figure et comprenant par exemple des moyens de fixation à baïonnette.

Différents moyens classiques et connus dans l'état de la technique peuvent être envisagés pour assurer un maintien en position de ces différentes pièces les unes par rapport aux autres en utilisation, comme par exemple des moyens de freinage, à franchissement de point dur, ...

On conçoit alors que cette brosse de prélèvement présente un certain nombre d'avantages par rapport à celles de l'état de la technique, en particulier en permettant de réaliser les deux portions de brosse, c'est-à-dire celle en forme de brosse et celle en forme d'écouvillon, de manière distincte et séparée pour leur conférer les caractéristiques notamment de longueur et de souplesse requises par chacune d'elles de manière spécifique.

Ceci permet alors d'améliorer les prélèvements effectués et la qualité des analyses correspondantes.

Bien entendu, d'autres modes de réalisation encore peuvent être envisagés.

## Revendications

1. Brosse de prélèvement cytologique du type comportant un manche de manipulation (2) à l'une des extrémités duquel sont prévues une première portion en forme de brosse de prélèvement exocol (4) et une seconde portion en forme d'écouvillon de prélèvement endocol (5) au centre de la première portion en forme de brosse (4), **caractérisée en ce que** la seconde portion en forme d'écouvillon (5) est fixée à l'extrémité du manche (2) et est détachable de la première portion par coulissement dans un trou (6) de la première portion en forme de brosse (4), muni de moyens (7) de retenue de cellules permettant de recueillir des cellules de la seconde portion en forme d'écouvillon (5), lors de son retrait de la première portion en forme de brosse (4).

2. Brosse selon la revendication 1, **caractérisée en ce que** la première portion en forme de brosse (4) est fixée sur le manche (2) par des moyens de fixation détachables (8).

3. Brosse selon la revendication 2, **caractérisée en ce que** les moyens de fixation détachables comprennent des moyens de fixation à baïonnette (8).

4. Brosse selon l'une quelconque des revendications précédentes, **caractérisée en ce que** les moyens de retenue (7) du trou de la première portion en forme de brosse (4) comprennent des moyens en forme de peigne à travers lesquels passe la seconde portion en forme d'écouvillon (5) lors de son retrait de la première portion en forme de brosse (4).

## Claims

1. Cytological sampling brush of a type comprising a handling sleeve (2), at one of the ends of which there are provided a first, brush-form, portion (4) for exocervix sampling and, in the centre of the first, brush-form, portion (4), a second, bottle-brush-form, portion (5) for endocervix sampling, **characterised in that** the second, bottle-brush-form, portion (5) is fixed to the end of the sleeve (2) and is detachable from the first portion by sliding in a hole (6) in the first, brush-form, portion (4), which hole (6) is provided with cell retention means (7) making it possible to collect cells from the second, bottle-brush-form, portion (5) when the latter is withdrawn from the first, brush-form, portion (4).

2. Brush according to claim 1, **characterised in that** the first, brush-form, portion (4) is fixed to the sleeve (2) by detachable fixing means (8).

3. Brush according to claim 2, **characterised in that** the detachable fixing means comprise bayonet fixing means (8).

4. Brush according to any one of the preceding claims, **characterised in that** the retention means (7) of the hole in the first, brush-form, portion (4) comprise comb-form means through which the second, bottle-brush-form, portion (5) passes when the latter is withdrawn from the first, brush-form, portion (4).

## Patentansprüche

1. Bürste für zytologische Probenentnahme der Art mit einem Bediengriff (2) an einem der Enden, an welchem ein erster Bereich in Form einer Bürste zur ExoCervix-Probenentnahme (4) und ein zweiter Bereich in Form einer Flaschenbürste zur Endo-Cervix-Probenentnahme (5) im Zentrum des ersten Bereiches in Bürstenform (4) vorgesehen sind, **dadurch gekennzeichnet, dass** der zweite Bereich in Form der Flaschenbürste (5) an dem Ende des Griffes (2) befestigt und von dem ersten Bereich durch Gleiten in einem Loch (6) in dem ersten bürstenförmigen Bereich (4) ablösbar ist, wobei das Loch mit Vorrichtungen (7) zum Rückhalten von Zellen ausgestattet ist, welche ermöglichen, Zellen von dem zweiten Bereich in Form einer Flaschenbürste (5) während seines Wegziehens von dem ersten bürstenförmigen Bereich (4) einzusammeln.

2. Bürste gemäß Anspruch 1, **dadurch gekennzeichnet, dass** der erste bürstenförmige Bereich (4) mit Hilfe von lösbaren Befestigungsvorrichtungen (8) an dem Griff (2) befestigt ist.

3. Bürste gemäß Anspruch 2, **dadurch gekennzeichnet, dass** die lösbaren Befestigungsvorrichtungen Vorrichtungen zur Bajonettbefestigung (8) aufweisen.

4. Bürste gemäß irgendeinem der vorherigen Ansprüche, **dadurch gekennzeichnet, dass** die Rückhaltevorrichtungen (7) des Loches des ersten bürstenförmigen Bereiches (4) kammförmige Vorrichtungen aufweisen, durch welche der zweite Bereich in Form einer Flaschenbürste (5) während seines Wegziehens von dem ersten bürstenförmigen Bereich (4) hindurch läuft.
